# FASCICULE DE BREVET EUROPEEN

(11) **EP 0 679 387 B1**
(45) Date de publication et mention de la délivrance du brevet: **09.04.1997**
(21) Numéro de dépôt: 95400583.1
(22) Date de dépôt: 16.03.1995
(51) Int. Cl.: A61K 7/48, A61K 7/06, A61K 31/375

(54) **Composition cosmétique et/ou dermatologique à support hydrophile et vitamine C mélangeables extemporanément**
Kosmetisches und/oder dermatologisches Mittel aus einem hydrophilen Träger und Vitamin C zum Mischen kurz vor Gebrauch
Cosmetic and/or dermatologic composition with a hydrophilic support and vitamin C for extemporaneously mixing

(30) Priorité: 05.04.1994 FR 9403982
(43) Date de publication de la demande: 02.11.1995
(73) Titulaire: L'OREAL, 75008 Paris (FR)
(72) Inventeur: Candau, Didier, F-91570 Bievres (FR); Collin, Nathalie, F-92330 Sceaux (FR); Quemin, Eric, F-93420 Villepinte (FR)
(74) Mandataire: Lhoste, Catherine

(56) Documents cités:
- EP-A- 0 399 157
- EP-A- 0 401 454
- WO-A-90/12572
- WO-A-93/11737
- DE-A- 3 642 097
- US-A- 4 711 780
- DATABASE WPI Week 9337, Derwent Publications Ltd., London, GB; AN 93-288819 & BR-A-9 004 902 (SOUKUP)

## Description

L'invention se rapporte à une composition contenant de l'acide ascorbique pulvérulent et un support hydrophile, conditionnés séparément et mélangeables juste avant l'emploi. Cette composition est utilisable dans les domaines cosmétique et/ou dermatologique par application topique sur la peau aussi bien du corps que du visage, y compris autour des yeux et sur le cuir chevelu.

L'invention se rapporte aussi à une utilisation de cette composition pour le traitement cosmétique de la peau ou pour la préparation d'un onguent pour un traitement dermatologique de la peau et des muqueuses, ainsi qu'à un procédé pour le traitement cosmétique de la peau.

On cherche depuis longtemps à formuler l'acide ascorbique ou vitamine C dans les domaines cosmétique et dermatologique, sous différentes formes galéniques, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter et/ou prévenir le vieillissement de la peau.

Malheureusement, en raison de sa structure chimique (d'alpha-cétolactone), l'acide ascorbique est très sensible à certains paramètres de l'environnement comme la lumière, l'oxygène, l'eau (par son pH et par la présence de traces de métaux). Il s'ensuit donc une dégradation rapide de l'acide ascorbique formulé en présence de ces paramètres, ce qui va à l'encontre de l'efficacité recherchée.

Pour diminuer et/ou retarder la dégradation de l'acide ascorbique, plusieurs solutions ont déjà été envisagées dans l'état de la technique.

L'une des solutions consiste à bloquer le site réactif de l'acide ascorbique, à savoir le site hydroxyle, notamment par estérification ou éthérification avec notamment des composés phosphatés, sulfatés ou alkylés (voir en particulier les documents US-A-5087446 et JP-A-69/115558 et JP-A-83/129892). Bien que plus stables que l'acide ascorbique, ces dérivés d'acide ascorbique sont malheureusement beaucoup moins efficaces que l'acide ascorbique.

Une autre solution consiste à formuler l'acide ascorbique dans un gel acide de pH au plus égal à 3,5 contenant une quantité élevée de glycol, comme décrit dans le document WO-A-90/12572. Du fait du pH acide utilisé et de la présence d'une grande quantité de glycol, c'est-à-dire de solvant, ce gel est mal toléré par la peau dont le pH est de 5,5 environ. Une application répétée de ce gel sur la peau peut entraîner des irritations.

Une autre solution consiste à utiliser l'acide ascorbique sous forme de poudre et à le dissoudre dans de l'eau juste avant l'emploi. Du fait de l'acidité élevée de la solution obtenue, celle-ci est agressive et risque de provoquer des irritations importantes de la peau.

Une autre solution utilise la lyophilisation d'une solution aqueuse contenant un ester de l'acide ascorbique et de l'acide hyaluronique ; le lyophilisât obtenu est dissous dans de l'eau, juste avant l'emploi. Cette solution présente les inconvénients d'être complexe et fort coûteuse ainsi que d'utiliser un dérivé de l'acide ascorbique, d'efficacité plus faible que celle de l'acide ascorbique. En outre, la solution obtenue est difficilement applicable sur la peau ; elle dégouline le long du visage et/ou du corps et est difficilement préhensible.

Dans le document FR-A-2645740, il est décrit une composition contenant une phase huileuse, une phase aqueuse et une phase solide à mélanger extemporanément, la phase solide renfermant un ou plusieurs actifs (notamment de la vitamine A) et un émulsionnant, et étant disposée dans le bouchon d'un flacon contenant les deux phases liquides. Ce conditionnement permet un stockage de longue durée de la composition avant le mélange des trois phases par agitation.

Après mélange, la composition n'est stable que quelques heures. De plus, le mélange obtenu est une émulsion grossière, peu homogène. Il s'ensuit une efficacité médiocre ; en effet, plus l'émulsion est grossière, moins elle est un bon véhicule d'actif. En outre, le solide se dissout mal. Par ailleurs, cette composition triphasée n'est nullement envisageable pour un actif à fonction acide, ce dernier risquant de casser l'émulsion, voire d'empêcher sa formation.

D'autres méthodes de stabilisation de l'acide ascorbique ont été envisagées et notamment son enrobage (technique décrite dans le document FR-A-1600826). Mais cette technique est d'une part coûteuse et peut, d'autre part, altérer l'acide ascorbique, par exemple lors d'un chauffage.

Par ailleurs, on connaît du document EP-A-399157, une composition topique pour les cheveux contenant un produit solide renfermant un acide et un carbonate ou bicarbonate, aptes à réagir ensemble, et un support fluide. Mais ce document enseigne la formation de mousse et par conséquent la présence impérative de carbonate ou bicarbonate destiné à réagir avec l'acide. Ce document n'enseigne pas d'utiliser l'acide seul en vue d'éviter sa décomposition par le support hydrophile. Le problème y est donc différent, de même que la solution proposée.

Il subsiste donc le besoin d'une composition cosmétique et/ou dermatologique contenant de l'acide ascorbique, pouvant être stockée plusieurs années avant d'être utilisée, ayant notamment les avantages d'être non irritante et efficace, tout en étant fabricable de façon simple et peu coûteuse, notamment en vue d'une production en grande série. De plus son emploi par l'utilisatrice est aisé.

Le demandeur a maintenant trouvé une nouvelle composition topique cosmétique et/ou dermatologique comportant un support hydrophile et un solide distincts, destinés à être mélangés extemporanément, le solide consistant essentiellement en de l'acide ascorbique pulvérulent et le support comprenant un milieu aqueux et au moins un gélifiant hydrophile, ce support étant capable de supporter l'introduction d'un composé acide.

Un support « capable de supporter l'introduction d'un composé acide » est un support qui ne casse pas et qui reste stable après l'introduction d'un composé acide.

A l'inverse de la composition du document EP-A-399157, le solide ne contient que de l'acide ascorbique pulvérulent et est exempt de carbonate ou de bicarbonate.

La composition selon l'invention est à mélanger avant emploi par simple secouement manuel. Le produit reconstitué est stable, à température ambiante, pendant au moins une semaine, ce qui n'est pas le cas du mélange obtenu selon le document FR-A-2645740. Par ailleurs, la dissolution de l'acide ascorbique dans le milieu aqueux se fait aisément, sans casser le support.

En outre, du fait que le support de l'invention incorpore de l'acide ascorbique à l'état protoné, ce support procure des traitements plus efficaces que ceux des préparations de l'art antérieur comportant des "dérivés" d'acide ascorbique.

Afin de faciliter l'introduction de l'acide ascorbique, le support doit être relativement fluide. Cette fluidité permet, en outre, de prendre du support par simple passage du doigt. En pratique, le support présente, avant mélange, une viscosité choisie dans la gamme de 1 à 25 poises (0,1 Pa.s à 2,5 Pa.s) et de préférence de 3 à 15 poises (0,3 Pa.s à 1,5 Pa.s).

Selon l'invention, on peut utiliser un ou plusieurs gélifiants hydrophiles utilisables dans les domaines cosmétique et/ou dermatologique, dont au moins un est capable de supporter l'introduction d'un composé acide. Ces gélifiants peuvent être choisis parmi les polysaccharides, les polymères synthétiques et les celluloses.

A titre d'exemple de gélifiants utilisables dans l'invention, on peut citer les gommes de guar, de xanthane, de carraghénane, de cellulose, les hydroxyalkylcelluloses, les carboxycelluloses de sodium, le Sépigel 305 et autres vendus par la société Seppic, les polyuréthannes, l'E-8/94 de la société Hoechst (acide polyacrylamidométhyl propane sulfonique), la gélatine, l'agar-agar, l'amidon.

La quantité de gélifiant est telle que le support, avant l'introduction de l'acide ascorbique, ait la viscosité indiquée précédemment.

Afin d'éviter l'obtention d'un support trop acide, c'est-à-dire de pH inférieur à 3,5, après introduction de l'acide ascorbique, il est préférable d'ajouter au support un ou plusieurs agents régulateurs de pH. A titre d'exemple, on peut utiliser un tampon au citrate de sodium ou à l'acétate de sodium. La quantité de tampon est fonction de la quantité d'acide ascorbique utilisé et du pH final recherché ; ce dernier est choisi avantageusement de 3,8 à 6 et de préférence de 3,8 à 4,5.

La quantité d'acide ascorbique est en pratique choisie de 0,1 % à 10 % du poids total de la composition, après mélange, et de préférence de 0,5 % à 8 % et encore mieux de 2 % à 6 %.

Le support hydrophile est de préférence un gel aqueux comportant éventuellement une dispersion de gouttelettes d'huile, avec ou sans émulsionnant, ces huile et émulsionnant étant tels que le support est toujours compatible avec l'introduction d'un composé acide. Comme gélifiant émulsionnant utilisable dans l'invention, on peut citer le Sépigel 305 et comme support émulsionné dans lequel on peut introduire un composé acide, on peut citer les émulsions aux dérivés de sucre telles que l'Arlatone 2121 de chez Ici. Ces émulsions ont l'aspect d'un lait ou d'une lotion.

Parmi les huiles utilisables dans l'invention, on peut citer indifféremment les huiles végétales, synthétiques, minérales, siliconées et/ou fluorées.

De façon avantageuse, et afin d'éviter notamment la présence dans la phase aqueuse de métaux lourds pouvant catalyser la dégradation de l'acide ascorbique, après mélange du support et du solide, la phase aqueuse est formée d'eau permutée ou désionisée.

Pour augmenter encore la stabilité de l'acide ascorbique au cours du temps, le support de l'invention peut comprendre, de plus, un agent séquestrant les métaux, tel qu'un dérivé d'acide phosphonique.

Les dérivés d'acide phosphonique utilisables dans l'invention sont notamment choisis parmi l'acide éthylènediamine tétra(méthylène phosphonique), l'acide hexaméthylènediamine tétra(méthylène phosphonique), l'acide diéthylènetriamine penta(méthylènephosphonique), et leurs sels et notamment leur sel de sodium comme le sel pentasodique de l'acide éthylènediamine tétra(méthylène phosphonique).

Par exemple, on utilise l'acide éthylènediamine tétra(méthylène phosphonique), vendu par la Société Monsanto sous la dénomination Dequest 2041. On peut aussi utiliser avantageusement le sel pentasodique de ce dernier qui est vendu sous la dénomination Dequest 2046 par la Société Monsanto.

La quantité d'agent séquestrant est en général au plus égale à 0,5 % du poids total de la composition et par exemple choisie de 0,05 % à 0,2 %.

Le support de l'invention peut contenir d'autres additifs que ceux cités précédemment, généralement utilisés dans les domaines cosmétique et dermatologique tels que par exemple les antioxydants (comme l'éthoxyquine pure pure vendue sous le nom de Raluquin par la Société Raschig), les émollients, les actifs hydrosolubles, les conservateurs (comme les parabens et le germal), les parfums, les matières colorantes (comme les pigments et les colorants hydrophiles) et les charges.

Les émollients sont en particulier des polyols comme les glycols (butylène glycol, isoprène glycol, propylène glycol et les polyéthylène glycols ayant de 4 à 6 moles d'oxyde d'éthyle par motif) et les glycérols. Ces émollients sont avantageusement présents à raison de 0 % à 5 % du poids total de la composition.

Les actifs hydrosolubles utilisables sont par exemple les eaux florales, les eaux thermales, les extraits de plante (d'aloès ou d'hamamélis servant d'anti-inflammatoire et d'adoucissant), le D-panthénol (servant d'anti-inflammatoire), l'hydroxyproline (servant d'actif réparateur et cicatrisant), l'urée et l'acide hyaluronique (servant d'hydratants). Ces actifs sont en général utilisés jusqu'à 1 % du poids total de la composition. Cependant les eaux florales ou thermales peuvent remplacer toute ou partie de l'eau du milieu aqueux.

Pour stocker la composition de l'invention, avant de mélanger le support et le solide, on peut utiliser deux récipients (ou flacons) séparés ou avantageusement un même récipient (ou flacon). En particulier, le support est conditionné dans un récipient fermé par un bouchon renfermant l'acide ascorbique, un séparateur perforable et/ou détachable étant prévu entre l'acide et le support. Ces conditionnements sont en particulier ceux décrits dans les documents FR-A-2476607, FR-A-2453793 ou FR-A-2645740.

Compte-tenu de la sensibilité de la vitamine C formulée, à la lumière, il est préférable d'utiliser un flacon opaque et/ou ambré, ne laissant pas passer la lumière.

L'acide ascorbique pulvérulent utilisable dans l'invention peut être celui vendu par Hoffmann-LaRoche; il se présente sous forme de l'acide L-ascorbique avec une pureté de 99% à 100%. Il serait bien entendu possible d'utiliser de l'acide D-ascorbique pulvérulent pur à 99% au moins.

La composition de l'invention peut être utilisée comme produit de soin de la peau et/ou comme produit dermatologique. En particulier, cette composition peut être utilisée pour lutter et/ou prévenir le vieillissement notamment en diminuant les ridules, en atténuant, voire en éliminant les taches apparaissant au cours du temps, en protégeant la peau des rayonnements U.V., en tonifiant la peau, en régénérant les tissus cutanés, et en donnant un éclat au teint.

La composition de l'invention peut aussi servir à cicatriser et/ou aseptiser des plaies.

Ainsi, l'invention a encore pour objet une utilisation de la composition définie ci-dessus pour un traitement cosmétique de la peau en vue de la tonifier, de la régénérer, de lisser les ridules de la peau, d'éclaircir le teint, d'atténuer les taches pigmentaires de la peau, et/ou pour lutter contre les méfaits des rayonnements U.V., et/ou pour renforcer les tissus cutanés contre les agressions de l'environnement.

L'invention a encore pour objet une utilisation de la composition précédente pour la fabrication d'un onguent destiné à un traitement dermatologique de la peau, comme la cicatrisation et l'aseptisation.

L'invention a encore pour objet un procédé de traitement cosmétique, caractérisé en ce qu'il consiste à appliquer sur la peau, y compris autour des yeux, une composition telle définie ci-dessus.

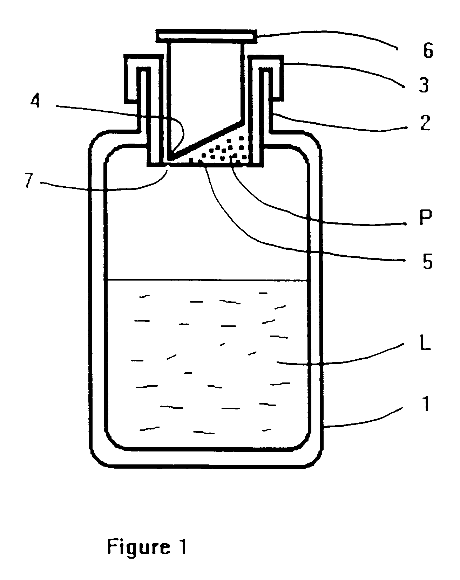

D'autres caractéristiques et avantages de l'invention ressortiront mieux de la description qui va suivre, donnée à titre illustratif et non limitatif en référence à la figure 1 annexée qui représente, en coupe longitudinale un flacon étanche à la lumière contenant la composition de l'invention, avant mélange du support (ici un gel) et du solide.

Le gel et le solide qui constituent la composition de l'invention, sont conditionnés dans un récipient à deux compartiments bien connus dans l'art antérieur. Le conditionnement montré sur la figure 1 est une vue simplifiée en coupe, d'un flacon bi-compartimenté. Il est constitué d'un corps (1) en un matériau faisant écran à la lumière extérieure, et faisant office de réservoir pour le gel fluide (L). A sa partie supérieure, le réservoir (1) est muni d'un col (2) dans lequel est inséré un godet (3), fermé à sa partie inférieure par un opercule (5), godet à l'intérieur duquel est placé le solide (P). L'opercule (5) peut être moulé en une pièce avec le godet (3) et comporte de préférence des zones très affaiblies (7).

En variante il est également possible de thermosouder un opercule en aluminium sur la partie inférieure du godet.

Dans la partie supérieure ouverte du godet (3) est insérée, de façon étanche, un trocard (6) muni à sa partie inférieure d'une zone tranchante (4) capable de découper une zone de l'opercule (5) lorsque l'utilisateur enfonce le trocard (6) vers le bas. La descente du trocard (6) provoque alors la déchirure de l'opercule (5) et le solide (P) peut s'écouler dans le gel fluide (L).

Avant utilisation, le godet (3) muni de son trocard (6) fait office de bouchon au récipient.

Avant l'application du produit, l'utilisateur agite cet ensemble pour dissoudre le solide constitué uniquement d'acide L-ascorbique pur à au moins 99 % dans le gel fluide et retire ensuite le trocard (6) du godet (3) pour libérer l'orifice de distribution. On peut éventuellement rajouter une tétine en plastique mou, percée à son extrémité, pour faciliter la libération du produit sur la peau.

On donne, ci-après, des exemples de compositions conformes à l'invention. Les quantités sont données en pourcentage pondéral.

### Exemple 1 : Gel à la vitamine C tamponné pH = 4

| **- *Gel :*** • *Phase A* | |
|---|---|
| - Carraghénane | 1,5 % |
| - Lubrajel (gélifiant) | 28,5 % |
| - Conservateur | 0,2 % |
| - Eau permutée | qsp 95 % |
| - Soude | 0,5 % |

| • *Phase B* | |
|---|---|
| - Eau permutée | 4,8 % |
| - Germall 115 | 0,3 % |
| - Dequest 2046 | 0,1 % |
| - Extrait d'aloès | 0,5 % |

| **- *Solide :*** | |
|---|---|
| - Acide ascorbique | 5 % |

Le gel est fabriqué de façon classique en préparant, séparément, par simple mélange les phases A et B respectivement à 70 °C et 40 °C, puis en ajoutant la phase B à la phase A sous agitation à 40 °C et en laissant refroidir l'ensemble sous agitation lente jusqu'à la température ambiante.

### Exemple 2: Gel à la vitamine C tamponné pH ≃ 4

| **- *Gel :*** • *Phase A* | |
|---|---|
| - Carraghénane | 1,3 % |
| - Guar | 0,5 % |
| - Lubrajel | 28,5 % |
| - Isoprène glycol | 4,8 % |
| - Conservateur | 0,2 % |
| - Eau permutée | qsp 95 % |
| - Soude } | 0,8 % |
| - Acide citrique } (régulateur de pH) | 1,2 % |

| • *Phase B* | |
|---|---|
| - Eau permutée | 4,8 % |
| - Germall 115 | 0,3 % |
| - Dequest 2046 | 0,1 % |
| - Extrait d'hamamélis dans le propylène glycol | 0,5 % |

| • *Phase C* | |
|---|---|
| - Colorant | 0,04 % |

| • *Phase D* | |
|---|---|
| - Parfum | 0,4 % |

| **- *Solide :*** | |
|---|---|
| - Acide ascorbique | 5 % |

Les phases A, B, C et D sont fabriquées séparément par simple mélange à respectivement 70 °C, 40 °C, 40 °C (ou 25 °C) et 40 °C (ou 25 °C). On introduit la phase B dans la phase A à 40 °C puis les phases C et D, sous agitation. On laisse l'ensemble revenir à la température ambiante, sous agitation lente.

### Test d'analyse sensorielle

La composition de l'exemple 2 a été testée sur un panel de 31 jurés pendant tout une journée.

Le gel a été jugé fluide et gélifié à la prise sur le doigt il tient suffisamment. A l'application, il est doux, peu gras et léger. Il pénètre facilement et ne savonne pas du tout.

Par ailleurs, les jurés ont noté des effets tenseur et filmogène.

L'opinion générale est bonne.

### Test d'utilisation

Un test d'utilisation pendant quatre semaines a été réalisé sur 14 femmes, dont 7 étaient agées de 30 à 49 ans et 7 autres agées de 50 à 70 ans. Ces femmes sont des expertes en produit de soin pour visage, dont la moitié avait la peau sèche et très sèche et l'autre moitié la peau grasse et mixte.

A la vue du produit, ces expertes ont jugé que les principes actifs seraient bien préservés et resteraint actifs tout le temps de la durée de l'utilisation ; les échantillons testés étaient destinés à un traitement d'une semaine.

Par ailleurs, elles ont estimé que le produit, après mélange des phases solide et gélifiée, présentait les propriétés cosmétiques suivantes :
- tonification / stimulation / régénération / hydration de la peau ;
- fermeté et finesse du grain de la peau ;
- lissage, douceur et atténuation des rides ;
- clarté et unification du teint, effet de bonne mine ;
- nutrition et confort des peaux sèches ;
- texture fraîche et légère.

### Test de stabilité

La stabilité de la composition de l'exemple 2 et celle d'une composition 3, identique à la composition de l'exemple 2 à l'exception d'un pH de 3 et donc d'une plus grande quantité d'acide citrique, ont été testées juste après mélange du gel et du solide et 8 jours après.

Les résultats obtenus sont portés dans le tableau ci-après pour 3 échantillons de chacune de ces compositions.

| Composition | Echantillon Numéro | Taux Théorique | **TENEUR** | | | |
|---|---|---|---|---|---|---|
| | | | T0jour | (Moyenne) | T8jours | (Moyenne) |
| **n° 3** | a | 5 % | 5,05 % | Flacon endommagé | Surdosage dû au flacon non étanche | |
| | b | 5 % | 4,98 % | (4,81 %) | 4,83 % | (4,69 %) |
| | c | 5 % | 4,64 % | | 4,54 % | |
| **n° 2** | d | 5 % | 4,79 % | (4,80 %) | 4,35 % | (4,37 %) |
| | e | 5 % | 4,72 % | | 4,60 % | |
| | f | 5 % | 4,89 % | | 4,15 % | |

D'après ce tableau, on constate que l'acide ascorbique est légèrement plus stable dans la composition la plus acide. Cependant, la perte en acide ascorbique dans la composition de l'exemple 2 n'est que de 9 % au bout de 8 jours.

## Revendications

1. Composition topique cosmétique et/ou dermatologique comportant un support hydrophile et un solide distincts, destinés à être mélangés extemporanément, le solide consistant essentiellement en de l'acide ascorbique pulvérulent et le support comprenant un milieu aqueux et au moins un gélifiant hydrophile, ce support étant capable de supporter l'introduction d'un composé acide.

2. Composition selon la revendication 1, caractérisée en ce que le support est un gel aqueux.

3. Composition selon l'une quelconque des revendications précédentes, caractérisée en ce que le support présente une viscosité choisie dans la gamme de 1 à 25 poises (0,1 Pa.s à 2,5 Pa.s).

4. Composition selon l'une quelconque des revendications précédentes, caractérisée en ce que l'acide ascorbique représente de 0,1 % à 10 % du poids total de la composition.

5. Composition selon l'une quelconque des revendications précédentes, caractérisée en ce que l'acide ascorbique représente de 2 % à 6 % du poids total de la composition.

6. Composition selon l'une quelconque des revendications précédentes, caractérisée en ce que le gélifiant est choisi parmi les polysaccharides, les polymères synthétiques et les celluloses.

7. Composition selon l'une quelconque des revendications précédentes, caractérisée en ce que le support renferme un agent régulateur de pH.

8. Composition selon l'une quelconque des revendications précédentes, caractérisée en ce que le support contient au moins un additif choisi parmi les séquestrants, les antioxydants, les émollients, les actifs hydrosolubles, les conservateurs, les parfums, les matières colorantes, les charges, les huiles et les émulsionnants.

9. Composition selon l'une quelconque des revendication précédentes, caractérisée en ce que le support est conditionné dans un récipient (1) fermé par un bouchon (3) renfermant l'acide ascorbique, un séparateur (5) perforable et/ou détachable étant prévu entre l'acide et le support.

10. Utilisation de la composition selon l'une quelconque des revendications 1 à 8, pour un traitement cosmétique de la peau en vue de la tonifier, de la régénérer, de lisser les ridules de la peau, d'éclaircir le teint, d'atténuer les taches pigmentaires de la peau, et/ou pour lutter contre les méfaits des rayonnements U.V., et/ou pour renforcer les tissus cutanés contre les agressions de l'environnement.

11. Utilisation de la composition selon l'une quelconque des revendications 1 à 8, pour la fabrication d'un onguent destiné à un traitement dermatologique de la peau.

12. Procédé de traitement cosmétique, caractérisé en ce qu'il consiste à appliquer sur la peau, y compris autour des yeux, une composition selon l'une quelconque des revendications 1 à 8.

## Claims

1. Cosmetic and/or dermatological topical composition containing a hydrophilic carrier and a solid separately, intended to be mixed immediately before use, the solid essentially consisting of powdered ascorbic acid and the carrier comprising an aqueous medium and at least one hydrophilic gelling agent, this carrier being capable of withstanding the introduction of an acidic compound.

2. Composition according to Claim 1, characterized in that the carrier is an aqueous gel.

3. Composition according to any one of the preceding claims, characterized in that the carrier has a viscosity chosen in the range from 1 to 25 poises (0.1 Pa.s to 2.5 Pa.s).

4. Composition according to any one of the preceding claims, characterized in that the ascorbic acid represents from 0.1 % to 10 % of the total weight of the composition.

5. Composition according to any one of the preceding claims, characterized in that the ascorbic acid represents from 2 % to 6 % of the total weight of the composition.

6. Composition according to any one of the preceding claims, characterized in that the gelling agent is chosen from polysaccharides, synthetic polymers and celluloses.

7. Composition according to any one of the preceding claims, characterized in that the carrier comprises a pH-regulating agent.

8. Composition according to any one of the preceding claims, characterized in that the carrier contains at least one additive chosen from sequestrants, antioxidants, emollients, water-soluble active agents, preservatives, perfumes, colorants, fillers, oils and emulsifiers.

9. Composition according to any one of the preceding claims, characterized in that the carrier is packaged in a container (1) closed with a cap (3) containing ascorbic acid, a perforable and/or detachable separator (5) being provided between the acid and the carrier.

10. Use of the composition according to any one of Claims 1 to 8, for a cosmetic treatment of the skin for toning it up, for regenerating it, for smoothing the wrinkles of the skin, for making the complexion lighter, for attenuating the pigmented spots of the skin and/or for combating the damage caused by UV radiation, and/or for strengthening the skin tissues against damage by environmental agents.

11. Use of the composition according to any one of Claims 1 to 8, for the manufacture of an ointment intended for a dermatological treatment of the skin.

12. Process of cosmetic treatment, characterized in that it consists of applying to the skin, including around the eyes, a composition according to any one of Claims 1 to 8.

## Patentansprüche

1. Topische kosmetische und/oder dermatologische Zusammensetzung, die einen hydrophilen Träger und einen Feststoff enthält, die voneinander getrennt vorliegen und die dazu vorgesehen sind, unmittelbar bei der Anwendung vermischt zu werden, wobei der Feststoff im wesentlichen aus pulverförmiger Ascorbinsäure besteht und der Träger ein wäßriges Medium und mindestens einen hydrophilen Gelbildner enthält und gegenüber dem Zusatz einer sauren Verbindung beständig ist.

2. Zusammensetzung nach Anspruch 1, dadurch gekennzeichnet, daß der Träger ein wäßriges Gel ist.

3. Zusammensetzung nach einem der vorhergehenden Ansprüche, dadurch gekennzeichnet, daß der Träger eine Viskosität im Bereich von 1 bis 25 P (0,1 bis 2,5 Pa·s) aufweist.

4. Zusammensetzung nach einem der vorhergehenden Ansprüche, dadurch gekennzeichnet, daß die Ascorbinsäure 0,1 bis 10 % des Gesamtgewichtes der Zusammensetzung ausmacht.

5. Zusammensetzung nach einem der vorhergehenden Ansprüche, dadurch gekennzeichetn, daß die Ascorbinsäure 2 bis 6 % des Gesamtgewichtes der Zusammensetzung ausmacht.

6. Zusammensetzung nach einem der vorhergehenden Ansprüche, dadurch gekennzeichnet, daß der Gelbildner unter Polysacchariden, synthetischen Polymeren und Celluloseverbindungen ausgewählt ist.

7. Zusammensetzung nach einem der vorhergehenden Ansprüche, dadurch gekennzeichnet, daß der Träger ein Mittel enthält, das den pH-Wert reguliert.

8. Zusammensetzung nach einem der vorhergehenden Ansprüche, dadurch gekennzeichnet, daß der Träger mindestens einen Hilfsstoff enthält, der unter Maskierungsmitteln, Antioxidantien, Emollentien, wasserlöslichen Wirkstoffen, Konservierungsmitteln, Parfums, Färbemitteln, Füllstoffen, Ölen und Emulgatoren ausgewählt ist.

9. Zusammensetzung nach einem der vorhergehenden Ansprüche, dadurch gekennzeichnet, daß der Träger in einem Behälter (1) abgepackt ist, der mit einem Stopfen (3) verschlossen ist, der die Ascorbinsäure enthält, wobei ein Separator (5) zwischen der Ascorbinsäure und dem Träger vorgesehen ist, der durchbohrt werden kann und/oder abreißbar ist.

10. Verwendung der Zusammensetzung nach einem der Ansprüche 1 bis 8 zur kosmetischen Behandlung der Haut zur Belebung der Haut, zur Regenerierung der Haut, zur Glättung von Hautfalten, zur Erzielung eines hellen Teints, zur Abschwächung von Pigmentflecken der Haut und/oder zur Bekämpfung schädlicher Folgen von UV-Strahlung und/oder zur Stärkung des Hautgewebes gegen Umweltbelastungen.

11. Verwendung der Zusammensetzung nach einem der Ansprüche 1 bis 8 zur Herstellung einer Salbe, die zur dermatologischen Behandlung der Haut bestimmt ist.

12. Verfahren zur kosmetischen Behandlung, dadurch gekennzeichnet, daß es darin besteht, eine Zusammensetzung nach einem der Ansprüche 1 bis 8 auf die Haut, einschließlich der Hautpartien um die Augen, aufzutragen.
